# DEMANDE DE BREVET EUROPEEN

(11) **EP 2 166 484 A1**
(43) Date de publication de la demande: **24.03.2010**
(21) Numéro de dépôt: 08368018.1
(22) Date de dépôt: 19.09.2008
(51) Int. Cl.: G06F 21/24

(54) **Procédé d'accès à des données nominatives, tel qu'un dossier médical personnalisé, à partir d'un agent local de génération**

(71) Demandeur: SCP Asclépios, 98000 Monaco (MC)
(72) Inventeur: Barbat, Harvé, 06300 Nice (FR); Abdelali, Jabir, Marrakech (MA); Coudert, Patrick, 06190 Roquebrune Cap Martin (FR)
(74) Mandataire: Schuffenecker, Thierry

(57) **Abrégé**

Un procédé d'accès à un dossier informatique personnalisé, comportant des données de nature technique telle que des données médicales ainsi que des données nominatives hautement confidentielles. Le procédé comporte la mise en oeuvre d'un agent de génération du dossier informatique personnalisé (DMN) contenu dans un dispositif de stockage (20), telle qu'une clé biométrique USB. Le dispositif de stockage (20) comporte en outre un fichier de chiffrement/déchiffrement et une table (PLT) de correspondance de liens entre les données nominatives et un identifiant anonyme (IDA). La création du dossier informatique personnalisé comportant les données DMN met en outre en oeuvre :
- une base de données sur un premier serveur (DMA, 300) comportant uniquement des informations anonymes chiffrées au moyen de ladite clé de chiffrage et en relation avec ledit identifiant anonyme (IDA), à l'exclusion de toute information de caractère nominatif ;
- un ensemble de tables sur un second serveur (TSB, 400) comportant des données de mises à jour desdites tables (PLT), chiffrées au moyen de ladite clé de chiffrage;
- une base de données documentaires sur un troisième serveur (GED 500), comportant des pièces jointes contenues dans ledit dossier personnalisé, indexé via ledit identifiant anonyme (IDA) et chiffrées au moyen de ladite clé de chiffrage contenue dans ledit dispositif de stockage (20).

## Description

### Domaine technique de l'invention

La présente invention concerne les systèmes d'informations, et notamment un procédé d'accès à un dossier informatique personnalisé, comportant des données techniques et des données nominatives.

### Etat de la technique

Les systèmes d'information sont aujourd'hui dotés de techniques particulièrement sophistiquées pour assurer la sécurité et la confidentialité des données, notamment lorsqu'elles touche des données sensibles comme des informations nominatives d'usagers.

Comme cela est connu, on sécurise les systèmes et les accès par un emploi systématique de mots de passe, de procédés de chiffrement ou cryptage de fichiers et l'on sécurise les accès aux réseau par des techniques spécifiques, tel que le protocole HTTPS pour le réseau Internet et WEP (*Wireless Encryption Protoco*/) pour les réseaux sans fils.

En dépit de l'existence et de l'efficacité de ces techniques de sécurité, certaines applications, notamment médicales, font naître des besoins très spécifiques.

Le domaine médical est en effet un domaine tout à fait particulier où il est nécessaire, d'une part, de garantir la sécurité et la confidentialité des informations mais également, d'autre part, de permettre une certaine circulation de données dès lors que ces données ne sont plus nominatives.

Comme on le sait, la relation particulière entre un patient et son médecin est spécifiquement protégée par le secret professionnel, lequel est absolu, et dont les atteintes sont sanctionnés de manière sévère dans la plupart des pays.

Ce secret professionnel couvre bien évidemment le dossier médical personnel du patient, lequel doit être verrouillé autant que faire ce peut.

Pour autant, ce secret médical ne doit toutefois pas empêcher la communication du dossier par des moyens électroniques modernes, non seulement dans le but de satisfaire l'intérêt particulier de son titulaire, mais aussi et dans une moindre mesure, de permettre une certaine « communication » dans un but d'intérêt plus général.

Sur le plan de l'intérêt particulier tout d'abord, on rappelle évidemment que, à l'image du patient contemporain qui devient nomade, il est nécessaire de permettre une communication du dossier au moyens de moyens modernes de communication afin de permettre, notamment, à un patient qui se déplace sur la planète, d'accéder à tout moment et en tous lieux à son dossier.

Sur le plan de l'intérêt général ensuite, il convient d'observer que certains éléments d'un dossier médical - notamment les éléments de pathologie, de diagnostique et de traitement à l'exclusion de tout élément nominatif - peuvent présenter un grand intérêt pour la communauté des praticiens et professionnels de la Santé. Les praticiens éprouvent le besoin, dans le souci de faire avancer les règles de l'art et la pratique médicale, de discuter et débattre des aspects cliniques et médicalisés de « leurs » patients. D'une manière plus générale, la recherche médicale - qui est le garant de la médicale de qualité qui se construit jour après jour, se nourrit de tous les éléments d'ordre statistique et collectif qui peuvent être échangés au sein de la collectivité des professionnels.

Par conséquent, sous l'éclairage des observations qui précèdent, on voit que le traitement des données médicalisées requiert des techniques toutes spécifiques pour tenir compte de cette double exigence :
1. garantir de manière absolue le respect du secret professionnel qui couvre la relation particulière entre le patient et son médecin, et
2. permettre une certaine « diffusion » de certains éléments - non nominatifs et parfaitement anonymes - dans le but de servir de support à un traitement collectif des données et de servir plus généralement la recherche médicale.

Il est donc souhaitable de disposer d'un outil spécifique, parfaitement adapté à un tel besoin et dilemme, en permettant d'organiser un traitement spécifique de ces données hautement sensibles.

Tel est le problème technique à résoudre par la présente invention.

### Exposé de l'invention

La présente invention a pour but de proposer un procédé de traitement et de suivi d'un dossier informatique personnalisé, comportant des données de nature techniques notamment médicales, et d'autres données nominatives hautement confidentielles.

La présente invention a pour but de proposer un procédé de traitement et de suivi de données médicalisées assurant une parfaite confidentialité des données tout en permettant aux patients d'accéder à distance à leur dossier, et ce en dépit de l'intervention de tiers, même jugés dignes de confiance.

Un autre but de la présente invention consiste à proposer un procédé de traitement et de suivi de données médicalisées permettant d'obtenir un accès anonyme de certains éléments des dossiers personnalisés, tout en garantissant un parfait anonymat sur les données extraites.

C'est un autre but de la présente invention que de fournir un procédé de traitement et de suivi de données médicalisées, à usage pour les praticiens, permettant de garantir à ces mêmes praticiens une maîtrise parfaite de l'anonymat de leurs patients même lorsque la gestion informatique fait intervenir des serveurs extérieurs gérés par des tiers de confiance.

L'invention réalise ces buts au moyen d'un procédé d'accès à un dossier informatique personnalisé, comportant des données de nature technique telle que des données médicales ainsi que des données nominatives hautement confidentielles. Le procédé comporte la mise en oeuvre d'un agent de génération du dossier informatique personnalisé (DMN) contenu dans un dispositif de stockage. Le dispositif de stockage comporte en outre un fichier de chiffrement/déchiffrement et une table (PLT) de correspondance de liens entre les données nominatives d'un patient et un identifiant anonyme (IDA). La création du dossier informatique personnalisé comportant les données DMN met en outre en ouvre :
- une base de données sur un premier serveur (DMA, 300) comportant uniquement des informations anonymes chiffrées au moyen de ladite clé de chiffrage et en relation avec ledit identifiant anonyme (IDA), à l'exclusion de toute information de caractère nominatif ;
- un ensemble de tables sur un second serveur (TSB, 400) comportant des données de mises à jour desdites tables (PLT); chiffrées au moyen de ladite clé de chiffrage;
- une base de données documentaires sur un troisième serveur (GED 500), comportant des pièces jointes contenues dans ledit dossier personnalisé, indexé via ledit identifiant anonyme (IDA) et chiffrées au moyen de ladite clé de chiffrage contenue dans ledit dispositif de stockage (20).

Dans un mode de réalisation préféré, le dispositif de stockage détenu par le praticien est un support de stockage externe offrant un haut niveau de sécurité sur les données qui y sont stockée.

Dans un mode de réalisation spécifique, la clé de chiffrage est crée de manière aléatoire lors de l'installation dudit agent DMN dans le premier dispositif de stockage externe utilisé.

Dans un mode de réalisation particulier, l'installation du premier dispositif de stockage comporte les étapes suivantes :
- vérification du mot de passe du praticien ;
- génération aléatoire d'une clé de chiffrement stockée dans ledit dispositif de stockage ;
- saisie/importation d'une liste nominative de patients gérée par le praticien considéré,
- transmission d'une requête audit premier serveur DMA dans le but d'obtenir le téléchargement d'une liste d'identifiants anonymes IDA correspondant à la liste nominative stockée localement,
- création de la première table de liens PLT intégrant les informations nominatives ainsi que les identifiants anonymes connus du serveur DMA ;
- chiffrement de ladite table de liens PLT au moyen de la clé de chiffrement générée aléatoirement.

Suivant un autre aspect de l'invention, il est prévu une procédure de duplication/qualification d'un dispositif de stockage source en vue de la création/qualification d'un second dispositif de stockage permettant la génération du dossier médical personnalisé et l'accès aux données nominatives (DMN).

A cet effet, l'agent DMN du dispositif de stockage primaire effectue les étapes suivantes :
- vérification du mot de passe du titulaire du premier dispositif de stockage servant de source à la duplication ;
- vérification de la présence de la table chiffrée PLT et du fichier comportant la clé de chiffrage ;
- vérification du mot de passe du titulaire du dispositif de stockage secondaire ;
- création sur ledit dispositif de stockage secondaire des fichiers comportant le fichier exécutable de l'agent DMN, la table de liens chiffrée PLT ainsi que le fichier de la clé de chiffrement utilisée par le dispositif de stockage primaire ;

Suivant un mode de réalisation préféré, la création/qualification de tout dispositif de stockage aboutit à la l'édition d'une attestation et/ou d'un certificat électronique.

De préférence, la mise à jour des dispositifs de stockage appartenant à un même groupe (et relevant de la même clé de chiffrement) est réalisée au moyen d'une procédure comportant les étapes suivantes :
- saisie et cryptage de données nominatives modificatives ou relatives à un nouveau patient ;
- transmission d'une requête sur le second serveur TSB en vue du stockage provisoire, sous une forme chiffrée au moyen de la clé de chiffrement contenue dans ledit dispositif de stockage (20), des informations nominatives à l'exclusion de l'identifiant anonyme (IDA) ;
- mise à jour de la table locale (PLT) intégrant les nouvelles informations modificatives ;
- chiffrement de la table locale (PLT) au moyen de la clé de chiffrement.

Afin de mettre à jour sa propre table PLT, un dispositif de stockage conforme à la présente invention effectue les étapes suivantes :
- vérification du mot de passe;
- vérification de la présence du fichier comportant la clé de chiffrement et de la table de liens PLT ;
- génération d'une requête transmise vers le premier serveur DMA en vue de l'obtention de la liste des identifiants anonymes stockés sur ce serveur ;
- identification de la liste des identifiants anonymes (IDA) téléchargés depuis ledit premier serveur DMA;
- déchiffrement de la table de liens locales PLT ;
- comparaison de la liste des identifiants anonymes téléchargés avec celle stockée dans la table PLT et, en cas de non concordance ;
- génération d'une requête à destination dudit second serveur TSB dans le but de télécharger les informations nominatives qui y sont stockées temporairement ;
- mise à jour de la table locale de liens PLT aux moyens des informations téléchargées depuis le second serveur TSB.
- vérification de la mise à jour de tous les dispositifs de stockage d'un même groupe et purge, le cas échéant, des données stockées sur le second serveur TSB.

Enfin, suivant un mode de réalisation particulier, le procédé comporte la mise en oeuvre d'un serveur administrateur permettant la gestion des licences ainsi que des purges dudit second serveur lorsque toute la mise à jour de tous les dispositifs de stockage appartenant à un même groupe ont été effectués.

L'invention est particulièrement adaptée au suivi et à la mise en ligne des données médicales des sportifs professionnels et Athlètes de haut niveau.

### Description des dessins

D'autres caractéristiques, but et avantages de l'invention apparaîtront à la lecture de la description et des dessins ci-après, donnés uniquement à titre d'exemples non limitatifs. Sur les dessins annexés :
La figure 1 illustre l'architecture générale d'un mode de réalisation d'un procédé de traitement et de suivi de données médicales.
Les figures 2A, 2B et 2C illustrent un exemple d'organisation des données au sein du serveur DMA 300
La Figure 3 illustre plus particulièrement le contenu du serveur TSB 400 servant au stockage temporaire (sous une forme encryptée) des données de mises à jour par le praticien.
La figure 4 illustre la structure de la table de liens PLT 21 qui est stockée dans chacun des supports externes protégés.
La figure 5 illustre le procédé permettant l'installation d'un premier dispositif de stockage 20 chez un praticien appartenant à un groupe professionnel, un organisme considéré.
La figure 6 illustre le procédé, mis en oeuvre par l'agent DMN, de duplication d'un dispositif de stockage 20-1 primaire, en vue de la qualification d'un dispositif de stockage secondaire 20-2
La figure 7 illustre le procédé permettant d'ajouter/modifier des données nominatives relatives à un athlète.
La figure 8 illustre le procédé mis en oeuvre pour procéder à la mise à jour des tables PLT dans les dispositifs de stockages 20-n.
La figure 9 illustre le procédé de construction du dossier DMN par l'agent DMN.

### Description d'un mode de réalisation préféré

L'on décrit à présent un mode de réalisation de l'invention permettant, d'une part, une gestion informatique et parfaitement sécurisée de dossiers médicaux par des professionnels de la santé exerçant en général dans le cadre d'un groupement professionnel.

L'invention est particulièrement adaptée à l'intervention de prestataires professionnels des systèmes d'information, et apporte toute garantie de confidentialité sur les données, médicales et nominatives, contenus dans les dossiers.

Plus spécifiquement, on considère dans le mode de réalisation qui va être décrit le traitement de données médicales de sportifs professionnels et athlètes de haut niveau. Les données médicales relatives à ces sportifs et athlètes sont particulièrement sensibles et exigent un haut niveau de confidentialité pour s'assurer l'absence de fuite. On sait en effet, qu'avec l'essor du pari en ligne, des tentatives de fraudes sont réelles et il importe de pouvoir sécuriser de manière particulièrement soignée les données nominatives de ces « consommateurs spécifiques » de soins médicaux...

Dans un tel contexte, les athlètes de haut niveau sont naturellement regroupés en groupements (fédérations, ligues, clubs etc.) auxquels sont rattachés un ensemble de praticiens professionnels (médecins, kinésithérapeutes etc...) exercent eux-même au sein d'un groupement professionnel.

L'invention va être décrite par rapport à un tel contexte afin d'illustrer la grande efficacité des procédés décrits. Pour autant, il convient de faire observer qu'il ne s'agit que d'un mode de réalisation particulier, permettant de décrire l'organisation des moyens qui sont mis en oeuvre et les avantages techniques qui en résultent. Un homme du métier pourra bien évidemment adapter l'enseignement qui suit à la mise en place d'un système plus général de suivi informatique de données médicales de patients quelconques.

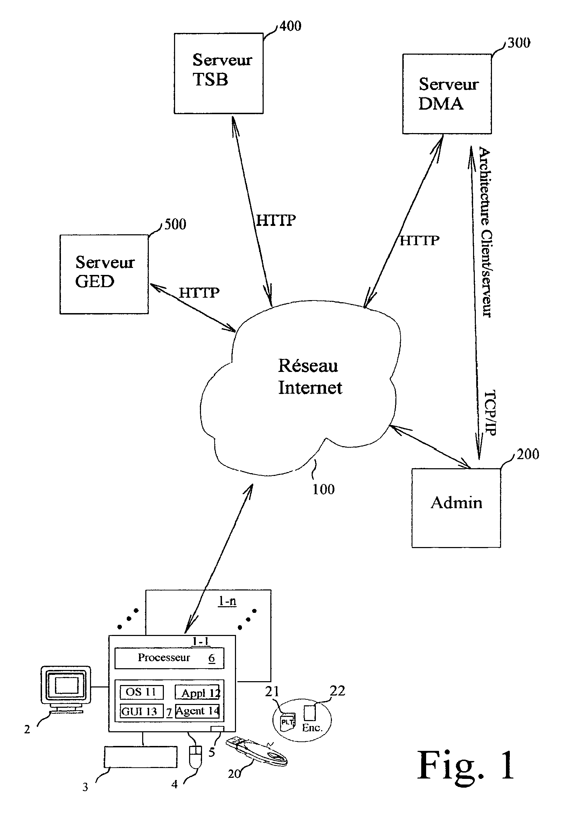

La figure 1 illustre l'architecture générale d'un mode de réalisation particulier. Chacun des praticiens au sein d'un même groupement de professionnels disposent d'un système informatique quelconque, tel qu'un ordinateur PC par exemple 1-1 à 1-n (on suppose que le groupement considéré comporte n systèmes ), doté d'un processeur 6, de moyens de stockage dont une RAM 7 dans laquelle se trouve chargé un système d'exploitation 11 (tel que WINDOWS (marque déposée) ou LINUX par exemple), un logiciel applicatif 12, un module d'interface utilisateur 13 ainsi qu'un agent DMN 14 pour la mise en oeuvre du procédé qui sera décrit ci-après. Le système 1-1 est doté en outre de moyens d'entrée/sortie conventionnels permettant la connexion à un écran 2, un clavier 3, un dispositif de pointage tel qu'une souris 4, ainsi que des ports spécifiques pour la connexion des périphériques adéquats, par exemple un périphérique série de type USB (*Universal Serial Bus*) ou IEEE1394 (*firewire*)*...*

Dans un mode de réalisation préféré, le système 1-1 dispose d'au moins d'un port série 5 destiné à recevoir un dispositif de stockage 20, prenant la forme d'un support externe protégé.

Chacun des praticiens exerçant au sein du groupement professionnel considéré disposera ainsi de son propre support externe de sauvegarde lequel sera chargé, d'un certains nombres de fichiers destinés à la mise en oeuvre des techniques et procédés qui seront décrits ci-après.

Chacun des systèmes 1-1 à 1-n dispose en outre de moyens de communication, notamment avec le réseau Internet 100 de manière à pouvoir accéder, par exemple via le protocole http (*Hyper Text Transfer Protocol*) ou tout protocole équivalent, à des serveurs extérieurs, et en particulier à un serveur Administrateur 200, un serveur DMA 300 pour le stockage - crypté - de données médicales anonymes, un serveur TSB 400 pour le stockage - crypté - de données nominatives temporaires ainsi qu'un serveur GED 500 pour la Gestion de documents.

Il est à noter que le serveur 200 pourra également communiquer avec le serveur DMA par des moyens de communications de type TCP/IP suivant une architecture de communication client-serveur bien connue d'un homme du métier qu'il n'est pas besoin de développer plus avant.

Par ailleurs, les praticiens pourront également être équipés de systèmes de communications mobiles, tel qu'un ordinateur portable, un dispositif de type PDA (*Portable Document Assistant -* non illustré), voire même un téléphone mobile de dernière génération permettant l'échange de données via le réseau Internet.

D'une manière générale, dans le contexte de la présente description et afin de clarifier l'exposé qui va suivre, on adoptera les définitions suivantes :

**Données Médicales Nominatives** (DMN) : l'on vise sous cette appellation le dossier complet du patient, comportant aussi bien des informations administratives et surtout nominatives (nom, age, sexe, particularités, adresse, téléphone...) que des informations médicales (pathologies, éléments de diagnostique, traitements, etc...).

Comme on va le voir ci-après, pour garantir un haut niveau de sécurité, les données DMN ne sont jamais stockées sur les serveurs dans l'approche proposée qui est proposée dans présente invention. Ces données sont générées, uniquement sur demande du praticien, directement en local sur son système 1-1 au moyen du dispositif de stockage 20.

**Données Médicales Anonymes** (DMA). L'on vise, dans cette catégorie des informations uniquement médicales à l'exclusion de toute information à caractère nominatif, tel que le nom, l'adresse, les coordonnées téléphoniques etc... Ces données DMA, moins sensibles que les données complètes DMN sont stockées sur le serveur DMA 300 indexé par un identifiant non nominatif, désigné par IDA. Bien qu'elles soient moins sensibles que les données DMN, l'accès au serveur 300 est néanmoins protégé par un cryptage connu des seuls praticiens du groupement professionnel considéré et stocké au sein du dispositif de stockage 20f. En raison de la nature des informations stockées sur le serveur 300, ce dernier aura vocation à fournir des éléments de nature statistique permettant aux praticiens et aux professionnels de la Santé en général, d'accéder à des informations de nature collective sur les pathologies, les diagnostiques et les traitements des patients.

**Base de Stockage Temporaire** (TSB). Cette base comporte des informations cryptées permettant une gestion des mises jour des dispositifs de stockage 20 utilisées au sein du groupement professionnel considéré.

**Base de stockage de documents** (DSB) : Cette base contient des fichiers cryptés correspondant à des documents PDF-ou image JPG.

D'une manière générale, les serveurs 200, 300 et 400 stockent des informations qui sont cryptées au moyen de clés uniquement présentes dans les dispositifs de stockage 20 détenues par les seuls praticiens, à savoir les supports de stockage externe. En particulier, l'administrateur qui gère le serveur d'administration 200 ne détient pas, comme on le verra ci-après, les clés de décryptage.

Le dispositif de stockage 20 - à savoir le support externe dans l'exemple considéré - comporte un fichier d'un exécutable qui est un agent DMN de génération de données DMN à partir des informations éparses présentes sur les différents composants du réseau.

Le dispositif de stockage 20 comporte en outre un fichier comportant les clés publiques/privés permettant le chiffrement/cryptage et décryptage des informations téléchargées sur les serveurs 300-400-500 et stockées sur ces serveurs. On notera qu'un homme du métier pourra utiliser tout procédé de cryptage/décryptage connu pour mettre en oeuvre la présente invention.

Outre le fichier des clés de cryptage/décryptage et l'exécutable de l'agent DMN, le dispositif de stockage 20 comporte une table de liens dites PLT :

***Table de liens* (PLT):** Cette table sert de pivot pour les échanges entre les différents serveur comme on va le voir. En effet, se trouve regroupée dans cette table, et uniquement dans celle-ci, la correspondant des informations nominatives avec l'index IDA utilisé notamment par le serveur DMA 300.

Suivant un des aspects de l'invention, les données nominatives - hautement sensibles - et les données purement médicales (pathologies - diagnostique - traitements) - qui sont simplement sensibles - font l'objet d'un traitement différentié.

Les données nominatives sont stockées dans le dispositif de stockage 20 détenu par le seul praticien et sont particulièrement protégés par les moyens électroniques mis en oeuvre par les procédés de l'invention (clé de chiffrement, protection biométrique) lesquelles viennent soutenir la protection physique apportée par ce même praticien à son dispositif de stockage.

Les données simplement médicales sont stockées sur un serveur externe, et est protégé - dans une moindre mesure - par un système de chiffrement réalisé par une clé qui est stockée par le praticien.

Suivant un aspect de l'invention, l'agent DMN du dispositif de stockage 20, lorsqu'il est mis en oeuvre dans le système 1-1 du praticien, procède à des requêtes entre sa table locale PLT mais aussi les tables stockées sur les serveurs distants DMA 300, TSB 400 et GED 500, pour générer sur demande, au sein du cabinet du praticien, le dossier médical personnalisé d'un athlète donné.

Cette construction du dossier médical personnalisé se fait au moyen de la correspondance entre les données nominatives contenues dans le fichier PLT et les données médicales anonymes téléchargées depuis le serveur DMA 300, accompagnés des pièces jointes téléchargées depuis le serveur GED 500 au moyen du lien anonyme IDA.

Parce qu'il est construit sur demande, le dossier médical personnalisé n'est jamais stocké sur un serveur quelconque et son accès reste sous la maîtrise complète du praticien qui est, alors, le garant du respect de la confidentialité des données de son patient.

Les serveurs DMA 300, TSB 400 et GED 500 ne comportent que des données non nominatives et qui, de surcroît, sont chiffrées au moyen d'une clé de chiffrement détenue par le seul professionnel.

Par conséquent, les tiers qui sont appelés à gérer et héberger les serveurs 300-500 ne peuvent connaître des données stockées dans ces mêmes serveurs.

La confidentialité des données est ainsi garantie de manière particulièrement efficace, grâce à l'agent générateur du dossier médical personnel du patient.

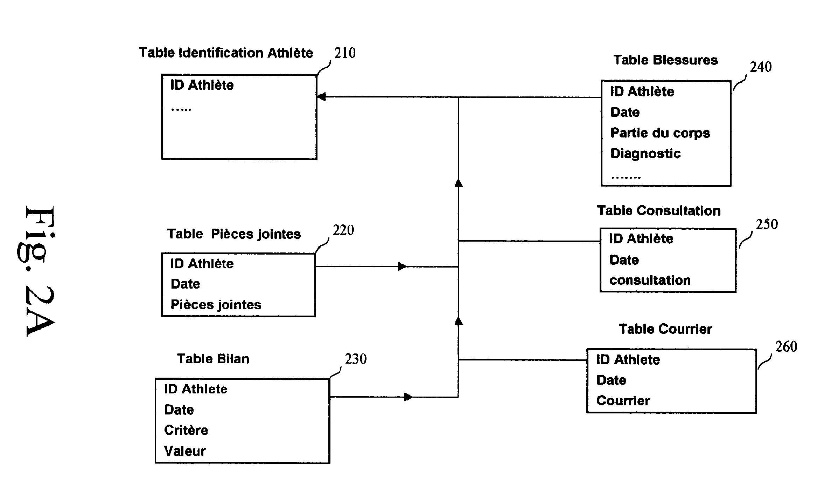

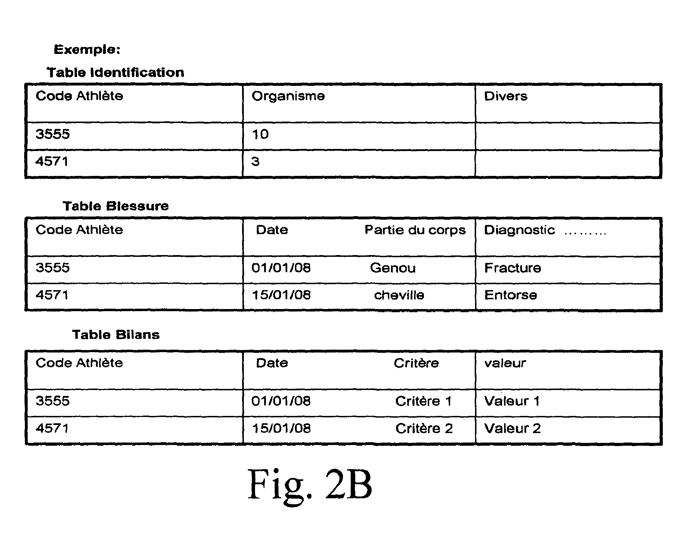

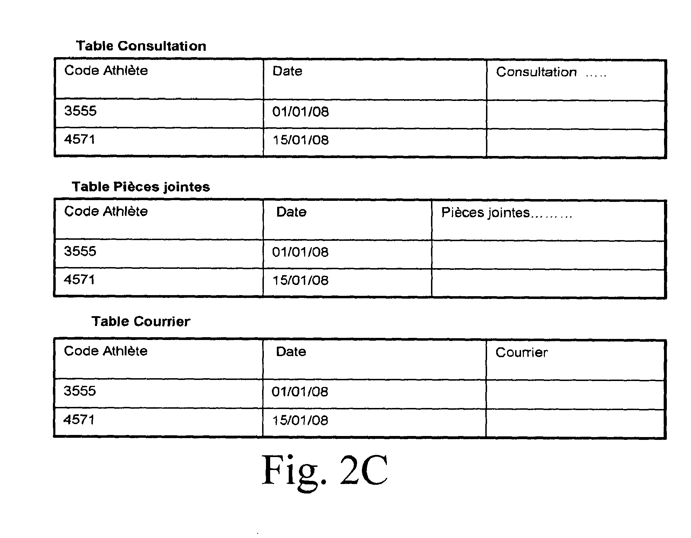

L'on décrit à présent, en relation avec la figure 2A, un exemple de structure de l'organisation des données au sein du serveur DMA 300. Comme on le voit, ce serveur comporte un ensemble de tables, dont les suivantes :

Table 210 : table d'identification des athlètes, laquelle n'est indexée qu'au moyen de l'identifiant anonyme IDA.

Table 220 : Table des pièces jointes. Cette table permet de regrouper les pièces jointes au dossier, convenablement datées, et stockées (sous une forme encryptée) sur le serveur GED 500.

Table 230: Table des bilans . Cette table permet de regrouper, à des instants choisis, les valeurs chiffrées des résultats cliniques obtenus sur le patient.

Table 240: Table des blessures. Cette table décrit l'historique des blessures ayant affecté le patient.

Table 250: Table des consultations Cette table collecte l'historique des consultations effectuées par le patient.

Tables des courriers. Cette table trace les date des échanges entre le patient et son praticien.

D'une manière générale, l'on constate sur les exemples illustrés dans les figures 2A-2C que les informations sont pour la plupart codifiées, ce qui prête particulièrement à un traitement statistique ultérieur ou à un suivi collectif de données médicalisées non nominatives. On notera que le terme « organisme » dans la table 2B renvoie naturellement au code du groupement de professionnels considéré (le club, la fédération, la ligue etc...), lequel est également codifié.

On notera en outre que l'invention se prête tout naturellement à l'utilisation systématique des codes issus de la classification CIM (Classification Internationale des Maladies) telle que publiée en son dernier état (CIM -10) par l'Organisation Mondiale de la Santé.

Dans le mode de réalisation particulier qui est considéré, à savoir un procédé de suivi de données médicales pour athlètes et sportifs professionnels de haut niveau, on pourra même développer des catégories et sous-classes spécifiques afin de rendre compte de certaines pathologies spécifiques propres au sport ou particulièrement intéressantes sur un plan d'étude statistique.

Comme on le voit le serveur 300 est appelé à constituer une base de données médicales, mais à l'exclusion de toutes données nominatives, susceptible de servir les besoins de suivi par les praticiens mais également à des études d'ordre statistique.

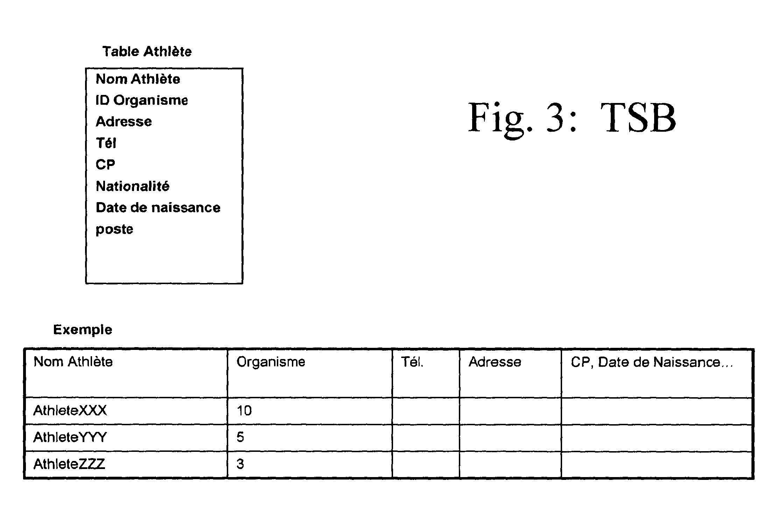

La Figure **3** illustre plus particulièrement le contenu du serveur TSB 400 servant au stockage temporaire (sous une forme chiffrée) des données de mises à jour par le praticien. Il est à noter, comme on le verra ci-après lors de l'exposé des protocoles de requêtes et des procédures exposées plus loin, que ce serveur TSB 400 a vocation à une purge périodique des données chiffrées, ce qui vient accroître la sécurité générale du procédé suivant l'invention.

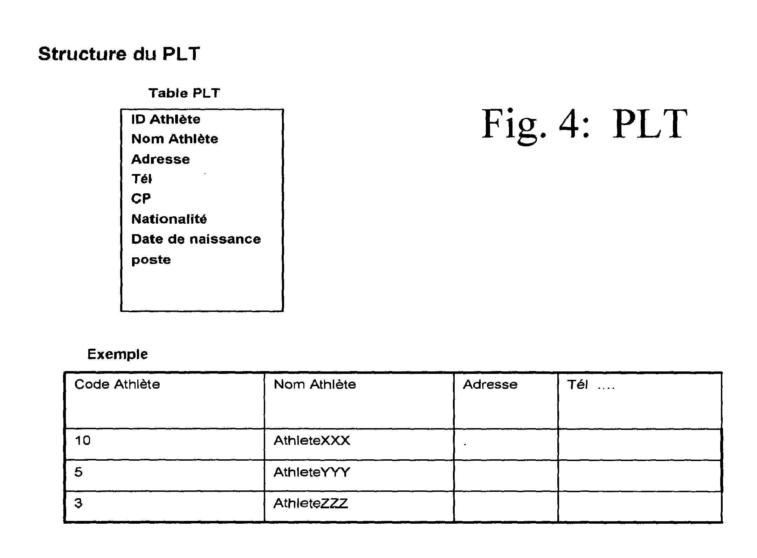

La figure **4** illustre la structure de la table de liens PLT 21 qui est stockée dans chacun des dispositifs de stockage 20, tels que par exemple les clés biométriques illustrées dans la figure 1.

Comme on le voit, cette table comporte, outre l'identifiant anonyme IDA, les éléments identification des athlètes, à savoir le nom, l'adresse, les coordonnées téléphoniques, la nationalité, ainsi que des éléments de profil associés à cet athlète tel que l'age, le sexe, des traits caractéristiques (droitier, gaucher etc...)

Comme on le voit, cette table PLT 21 présente des données hautement sensibles, et le procédé selon l'invention va procéder, comme on va le voir ci-après, à un traitement particulièrement sophistiqué de ces données afin d'assurer en permanence leur confidentialité, tout en permettant un certain traitement statistique et collectif sur des données médicales non nominatives.

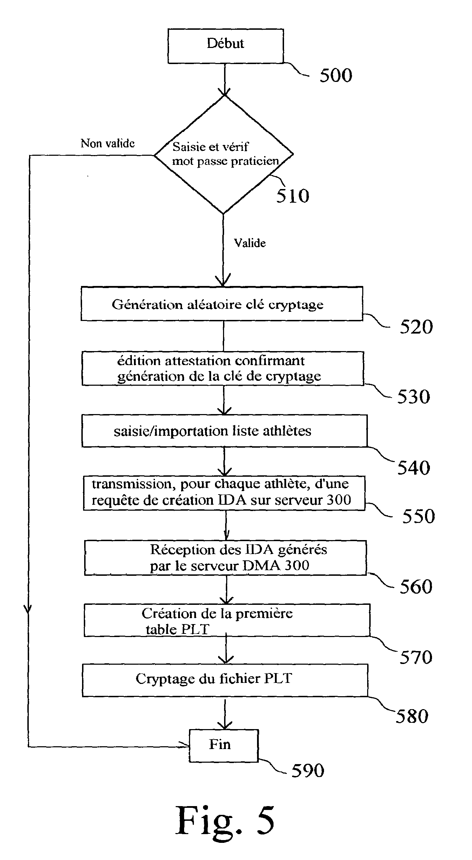

La figure 5 illustre le procédé permettant l'installation d'un premier dispositif de stockage 20 chez un praticien appartenant à un groupe professionnel, un organisme considéré.

Le procédé d'installation est basé sur la mise en oeuvre de l'agent DMN dont l'exécutable est stocké sur le dispositif de stockage 20. A cet effet, dans le cas d'une clé biométrique, il convient d'observer que, dès l'insertion de cette clé dans le port USB 5 du système, le praticien sera sollicité pour procéder à son activation. D'une manière générale, les principes et procédures mises en oeuvre pour permettre l'activation d'une clé biométrique - notamment par la capture de l'empreinte digitale une empreinte digitale - sont bien connues d'un homme du métier et ne font pas partie de la présente invention. L'on ne décrira pas plus en détail cette procédure d'activation et l'on se bornera à rappeler que celle-ci est basée sur une vérification de l'empreinte digitale présentée par le praticien et sa comparaison avec une empreinte de référence préalablement capturée et stockée au sein du dispositif 20.

On pourra avantageusement compléter le mécanisme de sécurité biométrique de tout autre mécanisme de sécurité, et notamment d'un mécanisme basé sur la capture d'une « empreinte » caractéristique de l'ordinateur, et de ses composants, sur lequel- se trouve connectée la clé biométrique.

Une fois l'activation de la clé biométrique effectuée, l'agent DMN peut s'exécuter et démarrer, par une étape 500, la procédure d'installation illustrée dans la figure 5.

L'agent DMN poursuit ensuite par une étape 510 de saisie et de vérification de mot de passe du praticien.

Si le test de l'étape 510 aboutit, le procédé poursuit avec une étape 520. Au contraire, si le test échoue, le procédé va directement à l'étape 590 mettant un terme à la procédure d'installation de la clé biométrique USB.

Dans l'étape 520, le procédé procède à une génération aléatoire d'une clé de cryptage qui, il faut le souligner, restera stockée uniquement dans le dispositif de stockage 20 chez le praticien, ainsi que dans les clés secondaires des confrères qui seront dupliquées comme on le verra ci-après.

D'une manière générale, on pourra envisager tout procédure de chiffrage/déchiffrage, et en particulier le procédé asymétrique de type RSA (Rivest, Shamir et Adleman) basé sur l'utilisation de clés publiques/privées et largement en usage sur Internet. On fixera également la longueur du code de chiffrement en fonction d'un niveau de sécurité que l'on souhaitera mettre en oeuvre pour la table PLT. A nouveau, les techniques de chiffrement, de codage sont bien connues d'un homme du métier et ne seront pas exposées plus en détail.

Le procédé poursuit ensuite par une étape 530 au cours de laquelle l'agent procède à l'édition d'une attestation ou d'un certificat (éventuel numérique) qui vient attester la génération de la clé de chiffrage/déchiffrage. Dans un mode de réalisation particulier, le procédé effectue une impression d'une attestation à faire signer par le praticien et visant à attirer son attention sur l'exigence de vigilance qui est attendu de lui afin de préserver la confidentialité des informations présentes sur la clé nouvellement installée.

Le procédé poursuit ensuite par une étape 540 visant à la saisie ou à l'importation d'une liste d'athlètes soit par saisie directe chez le praticien, soit au moyen d'une importation à partir d'un fichier préexistant sur le système sous la responsabilité du praticien. On pourra envisager que cette étape comporte également la saisie/importation de toutes les données nominatives des athlètes dans l'attente de la construction de la première table PLT.

Une fois la saisie opérée, le procédé poursuit par une étape 550 au cours de laquelle est générée, pour chacun des athlètes listés lors de l'étape précédente, une requête de création d'un nouvel IDA transmise au serveur DMA 300. Cela conduit à la création, sur ce serveur DMA 300, de l'environnement nécessaire à la constitution des tables indexées par les identifiants IDA nouvellement crées et qui seront ultérieurement mis à jour par les praticiens en fonction de leurs diligences auprès de leurs athlètes.

Dans une étape 560, l'agent DMN qui s'exécute sur le système du praticien récupère les IDA nouvellement crées en vue de la création de Ia structure de la première table PLT.

Dans une étape 570, l'agent procède ensuite, s'il ne l'a déjà fait lors de l'étape 540, à la saisie/importation des données nominatives des athlètes, mais également de données caractéristiques de leurs profils, afin de compléter la génération de la table PLT comme cela est illustré dans la figure 4. A nouveau, plusieurs modes de réalisations peuvent être envisagés, et notamment la saisie directe des informations nominatives par le praticien ou l'importation de ces mêmes données à partir d'un fichier préexistant sur le système du praticien. Il est important de constater que les données nominatives des athlètes demeurent au sein du cabinet du praticien, sous sa seule responsabilité, et ne sont nullement stockées sur les serveurs 200, 300, 400 ou 500.

Dans un mode de réalisation particulier, on peut prévoir d'interrompre par tout moyen quelconque la connexion entre le système du praticien et les serveurs 200-500 afin d'empêcher toute diffusion des données dans l'attente du cryptage ultérieur de la table PLT.

Le procédé se poursuit ensuite par une étape 580 au cours de laquelle l'agent effectue le cryptage, au moyen de la clé générée aléatoirement lors de l'étape 520, de la table PLT nouvellement crée.

Le procédé mis en oeuvre dans l'agent DMN s'achève ensuite par l'étape terminale 590.

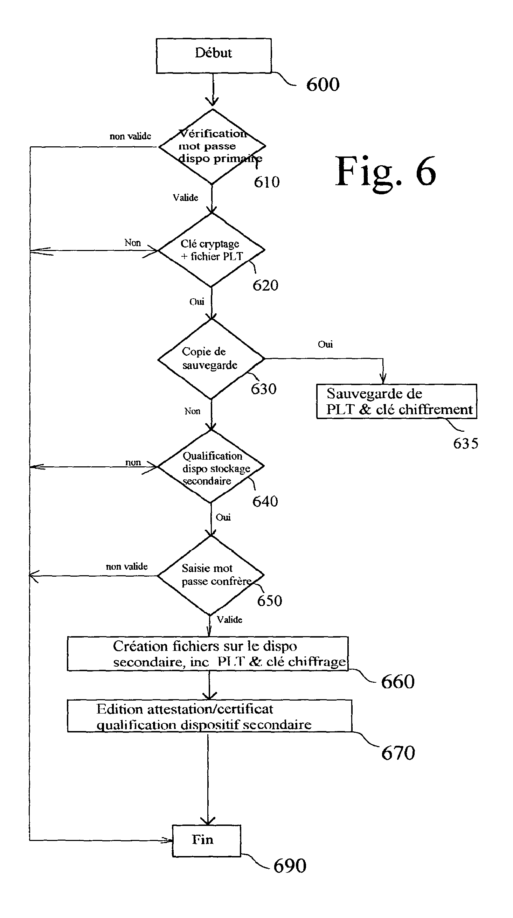

La figure **6** illustre le procédé, mis en oeuvre par l'agent DMN, de sauvegarde et/ou de duplication d'un dispositif de stockage 20-1 **source** ou **primaire**, en vue de la qualification d'un dispositif de stockage **destination** ou **secondaire** 20-2 permettant à un second praticien d'accéder au dossier DMN des athlètes suivi par le groupement professionnel.

Le procédé est mis en oeuvre sur le dispositif de stockage primaire démarre par une étape 600.

Puis le procédé poursuit par une étape 610 au cours de laquelle est mise en oeuvre une procédure de vérification du mot de passe du titulaire du dispositif primaire, semblable à la procédure décrite en relation avec l'étape 510 de la figure 5. Si le mot de passe n'est pas identifié comme étant valide, alors le procédé s'achève avec une étape 690.

Si la vérification du mot de passe est validée, alors le procédé va vers une étape 620, au cours de laquelle l'agent s'exécutant sur le dispositif primaire vérifie la présence du fichier de cryptage ainsi que de la table PLT cryptée.

Si l'un des deux fichiers est absent du dispositif de stockage primaire, alors le procédé va à l'étape 690 et s'arrête.

En revanche, si les deux fichiers attendus sont bien présents, alors le procédé poursuit avec une étape optionnelle 630 au cours de laquelle l'agent DMN détermine si le procédé doit simplement mener à une copie de sauvegarde du fichier PLT et du fichier de la clé de cryptage. Dans l'affirmative, le procédé va à une étape 635 où les deux fichiers sont stockés sur un support de stockage adéquat, sous la responsabilité du praticien.

Dans le cas contraire, ou lorsque l'étape 630 n'est pas prévue, le procédé va directement de l'étape 620 vers une étape 640 au cours de laquelle l'agent demande au praticien primaire de confirmer l'opportunité de procéder à la qualification d'un dispositif de stockage secondaire en vue de la création d'une seconde clé d'accès au dossier médical personnalisé et, dans le cas contraire, le procédé va vers l'étape finale 690.

Si le praticien primaire confirme la procédure de qualification du dispositif de stockage secondaire, alors le procédé va vers une procédure 650 où l'agent invite le praticien secondaire à venir insérer son propre dispositif de stockage 20-2. Dans l'hypothèse ou le dispositif secondaire est, lui aussi, une clé USB biométrique, le praticien secondaire va devoir activer cette dernière pour permettre l'écriture de fichiers et, par suite, la bonne marche de la procédure de qualification. L'étape 650 se poursuit également par une procédure de saisie/création de mot de passe secondaire, lequel sera utilisé par le praticien secondaire pour accéder à l'exécution de son propre agent DMN.

Si la procédure de saisie/création de mot de passe n'aboutit pas, le procédé va ensuite à l'étape 690 et s'arrête.

En revanche, si la procédure de création du mot de passe du praticien secondaire aboutit et est validée, alors l'agent DMN qui s'exécute sur le dispositif de stockage primaire 20-1 procède dans une étape 660 à la génération des fichiers nécessaires à l'exécution d'une nouvelle instance de l'agent DMN, à savoir le fichier exécutable de l'agent DMN, le fichier de chiffrement/déchiffrement ainsi que la table PLT chiffrée, venant compléter la qualification du dispositif de stockage secondaire 20-2 , à savoir la seconde clé biométrique USB qui sera utilisée par le second praticien.

Puis, dans une étape 670, l'agent primaire procède à l'édition d'une attestation ou d'un certificat électronique venant confirmer la qualification du dispositif de stockage secondaire 20-2..

Le procédé s'achève ensuite par l'étape 690 mettant un terme à la procédure de sauvegarde/duplication.

On notera, et ceci est un avantage significatif de la présente invention, que le procédé de duplication des clés et de qualification du dispositif de stockage secondaire peut également servir à un même praticien qui souhaiterait réaliser une seconde copie « physique » de sa clé USB. Dans ce cas, il lui suffira de saisir une nouvelle fois son mot de passe lors de l'étape 650.

Ainsi, comme on le voit, le procédé permet de gérer très simplement les dispositifs de stockage primaire, secondaire, tertiaire, etc ... qui sont susceptibles de servir au sein d'un même groupement professionnel. Il est très aisé, pour les praticiens d'un groupement, de réaliser leur propre copies ou duplication de ce dispositif de stockage qui, il faut le rappeler, est absolument nécessaire pour accéder aux données nominatives du Dossier médical personnel des athlètes... A nouveau, hors le cabinet professionnel des praticiens et hors leur présence, il n'est pas possible, même pour l'administrateur du serveur 200, d'accéder aux données nominatives.

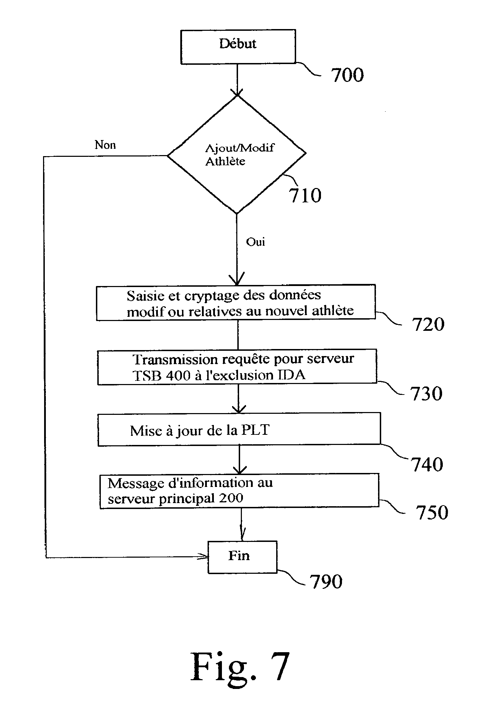

La figure 7 illustre le procédé permettant d'ajouter/modifier des données nominatives relatives à un athlète, et ce au moyen d'un dispositif de stockage 20-n.

Le procédé d'accès au DMN et de mise à jour démarre alors par une étape 700 . Comme précédemment, cette étape pourra comporte, outre l'étape préliminaire indispensable d'activation de la clé biométrique 20-n, de la saisie du mot de passe pour permettre l'exécution de l'agent DMN permettant la construction en ligne du DMN.

Dans une étape 710, un test est effectué pour déterminer s'il est nécessaire d'ajouter ou de modifier les informations concernant un athlète.

Dans la négative, le procédé va vers une étape terminale 790.

Dans l'affirmative, le procédé poursuit avec une étape 720 au cours de laquelle les données nominatives et de profil du nouvel athlète sont saisies/importées par le praticien et chiffrées au moyen de la clé de chiffrage présente sur le dispositif de stockage 20-n.

Puis dans une étape 730, le procédé poursuit avec la génération d'une requête transmise au serveur TSB 400, ladite requête comportant les informations de mises à jour saisies lors de l'étape 720, à toutefois de l'identifiant IDA.

Cela a pour avantage significatif de ne pas externaliser, hors le cabinet des praticiens, la table de correspondance entre le IDA et les éléments nominatifs d'identification des athlètes... Comme on l'a observé, la table stockée (pour un temps très limité) sur le serveur TSB 400 ne comporte que des données nominatives (à l'exclusion d'élément de nature médicale), tandis que le serveur DMA 300 ne comporte, lui, que des données médicalisées mais uniquement en relation avec un identifiant IDA non nominatif.

Et, de surcroîts, toutes les informations stockées sur les serveurs 300 et 400 sont chiffrées au moyen de clé uniquement détenues au sein du groupement professionnel des praticiens.

L'on peut bénéficie d'un degré de sécurité élevé et ce sans recourir à des techniques particulièrement coûteuses...

A la suite de l'étape 730, le procédé mis en oeuvre par l'agent DMN poursuit avec une étape 740 au cours de laquelle la table PLT est mise à jour en tenant compte de l'ajout/modification introduite par le praticien titulaire de la clé bio métrique primaire.

L'agent DMN transmet ensuite lors d'une étape 750 une requête au serveur principal 200 destiné à informer celui-ci de la mise à jour introduite dans le système. Il est à noter que seul l'identifiant IDA est transmis à cette occasion.

Le procédé s'achève ensuite par l'étape 790 qui finalise la modification intervenue au sein des tables.

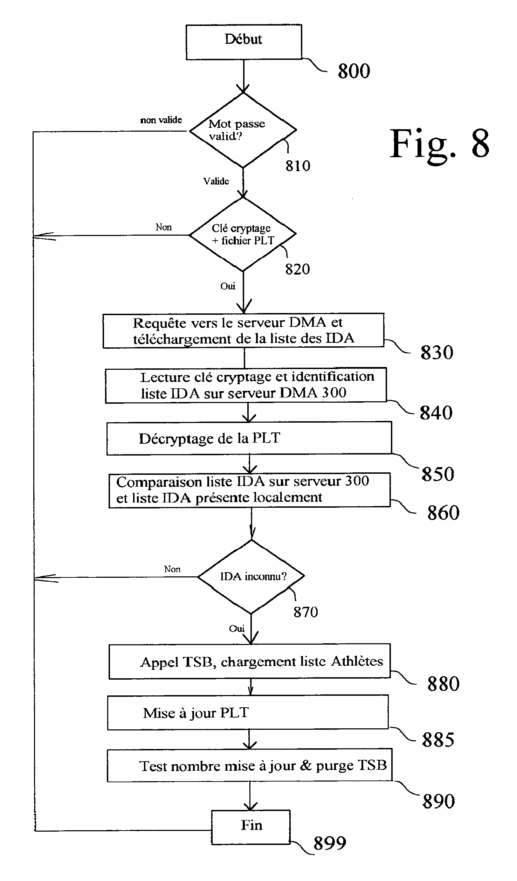

En référence à la figure 8, on décrit à présent le procédé mis en oeuvre pour procéder à la mise à jour des tables PLT dans les dispositifs de stockages 20-n etc...

Le procédé démarre par une étape 800. On suppose, comme précédemment, que le praticien qui a lancé l'exécutable de l'agent DMN sur son dispositif de stockage 20-n a satisfait à la procédure d'activation de sa clé biométrique.

Dans une étape 810, l'agent DMN lance la procédure de vérification du mot de passe qui, si elle échoue, renvoie directement à une étape finale 899.

Au contraire, si le praticien satisfait à la procédure de vérification de son mot de passe, l'agent DMN va à une étape 820 au cours de laquelle un test supplémentaire est effectué afin de déterminer la présence du fichier comportant la clé de chiffrement/déchiffrement ainsi que la table PLT.

Si le test échoue, le procédé va également à l'étape finale 899.

Si le test réussit, le procédé va alors à une étape 830 où l'agent génère une requête à destination du serveur DMA 300 dans le but de télécharger la liste des IDA.

Puis, dans une étape 840, l'agent procède à la lecture de la clé de déchiffrement pour obtenir la liste des IDA téléchargée depuis le serveur DMA 300.

Puis dans une étape 850, l'agent DMN procède au décryptage de sa table locale PLT pour pouvoir accéder aux données présentes sur cette dernière.

Dans une étape 860, l'agent DMN procède à une comparaison entre, d'une part, la liste des identifiants IDA téléchargés depuis le serveur DMA 300 et, d'autre part, la liste des IDA localement présente sur sa table PLT.

Dans une étape 870, l'agent DMN procède à un test pour déterminer si un IDA apparaît comme étant non attribué à l'un des joueur nommément identifié dans sa table PLT locale.

Si le test échoue, cela signifie qu'aucune mise à jour n'est nécessaire et le procédé va à l'étape finale 899.

Dans le cas contraire, le procédé identifie un ou plusieurs identifiant IDA non attribué, et va alors à une étape 880 pour transmettre une requête au serveur TSB 400 dans le but de télécharger la liste des athlètes.

Les informations nominatives présentes sur la base TSB 400 sont alors reçues localement par l'agent DMN qui, au moyen de sa clé de déchiffrage, peut y accéder et venir compléter sa table PLT locale, dans une étape 885.

Puis, l'agent procède à une notification transmise au serveur principal 200 dans le but d'informer ce dernier de la mise à jour intervenue. Ce dernier peut alors s'assurer que tous les dispositifs de stockage 20-n du même groupement professionnel ont bien été mis à jour et, le cas échéant, procéder à une purge de la table stockée dans le serveur temporaire TSB 400.

Le procédé s'achève enfin par l'étape 899.

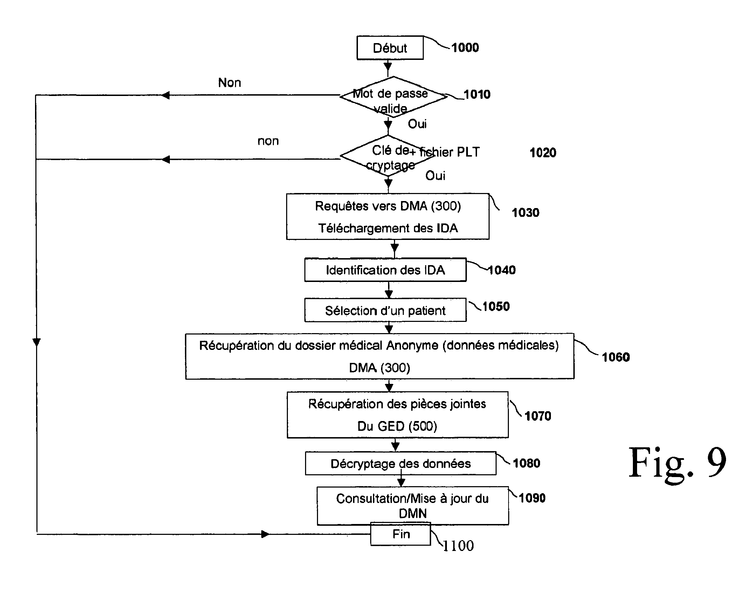

La figure 9 illustre le procédé de construction par l'agent DMN du dossier médical personnalisé comportant les données DMN .

Le procédé débute par l'étape 1000.

Puis, dans une étape 1010, l'agent DMN effectue un test de mot de passe pour vérifier que l'utilisateur est bien autorisé à provoquer la construction du dossier DMN et, dans le cas d'un mot de passe non valide, le procédé va à l'étape terminale 1100.

Si le mot de passe est reconnu comme étant valide, le procédé procède alors, dans une étape 1020, à la vérification de la présence du fichier PLT 21 et du fichier 22 comportant la clé de chiffrement sur le dispositif de stockage (par exemple la clé biométrique).

Si les deux fichiers ne sont pas simultanément présents, alors l'agent va à l'étape terminale 1100.

Si les deux fichiers sont présents, alors l'agent génère, dans une étape 1030, des requêtes à destination du serveur DMA 300 , et procède au téléchargement de la liste des identifiants anonymes IDA.

Dans une étape 1040, l'agent DMN procède à l'identification des patients au moyen de la table de correspondance stockée dans son dispositif de stockage sécurité.

Dans une étape 1050, l'agent DMN propose le choix d'une sélection d'un des patients identifiés à l'étape précédente.

Puis, dans une étape 1060, l'agent DMN procède au téléchargement des données anonymes depuis le serveur DMA 300 au moyen de l'IDA particulier attribué au patient considéré.

Puis, dans une étape 1070, l'agent DMN procède au téléchargement des pièces jointes stockées sur le serveur GED 500.

Dans une étape 1080, le procédé utilise la clé de chiffrement présente sur le dispositif de stockage sécurisé dont est titulaire le praticien afin de décrypter les données téléchargées depuis le serveur DMA 300 et le serveur GED 500.

Le dossier est à présent complet et peut être présenter de manière conviviable au moyen de l'Interface graphique utilisateur GUI 13 représentée dans la figure 1. Cette consultation s'effectue lors d'une étape 1090 permettant également au praticien de procéder à des éventuelles mises à jour et modifications du dossier de son patient, lesquelles mises à jour pourront être téléchargées vers leurs serveurs respectifs (DMA 300 notamment).

A la fin de la consultation, l'agent DMN va à l'étape terminale 1100 pour achever le procéder et effacer de la mémoire toute trace du dossier médical du patient.

Les procédés qui viennent d'être décrits permettent de créer, de manière sécurisée, des tables constitutives de bases de données médicalisées faisant l'objet de traitement techniques particulièrement complexes et garantissant, tout à la fois, un haut niveau de confidentialité et un traitement collectif et statistique de certaines données parfaitement anonymes.

En effet, comme on le voit, le dossier médical personnel d'un patient n'est crée que chez le praticien, et ce au moyen de l'agent 12 de génération des données DMN bénéficiant d'une double niveau de protection : la protection résultant du chiffrement avec la clé uniquement présente sur le dispositif de stockage 20, combinée à la protection résultant du mécanisme de vérification biométrique mis en oeuvre chez le praticien.

Par conséquent, hors l'enceinte du praticien, et sans le recours au dispositif de stockage 20 à protection biométrique, il n'est pas possible de reconstruire le dossier médical d'un patient à l'insu du praticien, et les serveurs, notamment le serveur DMA 300 ne comporte que des données non nominatives (et de surcroît chiffrées).

En revanche, il reste possible, pour un praticien du groupement professionnel considéré ou pour un praticien fédérant les groupements professionnels institués, d'accéder à certains éléments d'informations, tels que des codes issus de la classification CIM-10 de l'OMS par exemple, ou toute codification sous-jacente et/ou distincte, pour effectuer un traitement collectif de ces données voire, pour procéder à une communication non nominative de ces données.

Par les moyens techniques qu'elle met en oeuvre, l'invention permet ainsi de réaliser un lien particulièrement satisfaisant entre des données hautement sensibles, les données stockées dans la table PLT qui doivent rester absolument confidentielle, et des données susceptibles de permettre un traitement collectif dans le but de faire avancer le suivi des dossiers et/ou la recherche médicale.

## Revendications

1. Procédé d'accès à un dossier informatique personnalisé, comportant des données de nature technique, notamment des données médicales, et des données nominatives hautement confidentielles, **caractérisé en ce qu'**il comporte:
- la mise en oeuvre d'un agent de génération du dossier informatique personnalisé (DMN) dans au moins un dispositif de stockage (20), ledit dispositif de stockage comportant en outre un fichier de chiffrement/déchiffrement et une table (PLT) de correspondance de liens entre les données nominatives d'un patient et un identifiant anonyme (IDA),
- la mise en oeuvre d'une base de données sur un premier serveur (DMA, 300) comportant uniquement des informations anonymes chiffrées au moyen de ladite clé de chiffrage et en relation avec ledit identifiant anonyme (IDA), à l'exclusion de toute information de caractère nominatif ;
- un ensemble de tables sur un second serveur (TSB, 400) comportant des données de mises à jour desdites tables (PLT), chiffrées au moyen de ladite clé de chiffrage;
- la mise en oeuvre d'une base de données documentaires sur un troisième serveur (GED 500), comportant des pièces jointes contenues dans ledit dossier personnalisé, indexé via ledit identifiant anonyme (IDA) et chiffrées au moyen de ladite clé de chiffrage contenue dans ledit dispositif de stockage (20).

2. Procédé selon la revendication 1 **caractérisé en ce que** ledit dispositif de stockage est une clé biométrique de type USB.

3. Procédé selon la revendication 1 **caractérisé en ce que** ladite clé de chiffrage est crée de manière aléatoire lors de l'installation dudit agent DMN dans le premier dispositif de stockage (20).

4. Procédé selon la revendication 1 **caractérisé en ce que** l'installation du premier dispositif de stockage comporte les étapes suivantes :
- vérification (510) du mot de passe du praticien ;
- génération aléatoire (520) d'une clé de chiffrement stockée dans ledit dispositif de stockage (20) ;
- saisie/importation (540) d'une liste nominatives de patients,
- transmission (550) d'une requête audit premier serveur (DMA, 300) dans le but d'obtenir une liste d'identifiants anonymes IDA correspondant à la liste nominative stockée localement ;
- réception (560) dudit premier serveur (DMA, 300) de la liste d'identifiants anonymes (IDA) ;
- création (570) de la première table de liens (PLT, 21) intégrant les informations nominatives ainsi que les identifiants anonymes connus du serveur DMA ;
- chiffrement (580) de ladite table de liens (PLT, 21) au moyen de la clé générée dans l'étape 520.

5. Procédé selon la revendication 4 **caractérisé en ce qu'**il comporte, postérieurement à la génération aléatoire de la clé, la création d'une attestation/certificat permettant de confirmer la création de la clé de chiffrement.

6. Procédé selon la revendication 4 **caractérisé en ce qu'**il comporte une procédure de duplication/qualification d'un dispositif de stockage source (20) en vue de la création/qualification d'un second dispositif de stockage (20-n) permettant la génération du dossier médical personnalisé et l'accès aux données nominatives (DMN), ledit agent DMN du dispositif de stockage primaire effectuant les étapes suivantes :
- vérification (610) du mot de passe du titulaire du premier dispositif de stockage (20) servant de source à la duplication ;
- vérification (620) de la présence de la table chiffrée (PLT, 21) et du fichier de chiffrement (22) ;
- vérification (640, 650) du mot de passe du titulaire du dispositif de stockage secondaire ;
- création (660) sur ledit dispositif de stockage secondaire, des fichiers comportant le fichier exécutable de l'agent DMN, la table de liens chiffrée (PLT, 21), et du fichier comportant la clé de chiffrement utilisée par ledit dispositif de stockage primaire (20).

7. Procédé selon la revendication 6 **caractérisé en ce que** la création/qualification dudit dispositif secondaire comporte l'édition d'une attestation et/ou certificat.

8. Procédé selon la revendication 6 **caractérisé en ce qu'**il comporte les étapes suivantes destinées à la mise à jour des informations nominatives détenues dans les tables de liens des différents dispositifs de stockage (20) appartenant à un même groupe et utilisant la même clé de chiffrement :
- saisie et cryptage (720) de données nominatives modificatives ou relatives à un nouveau patient ;
- transmission (730) d'une requête sur le second serveur (TSB , 400) en vue de leur stockage provisoire, sous une forme chiffrée au moyen de la clé de chiffrement contenue dans ledit dispositif de stockage (20), des informations nominatives à l'exclusion de l'identifiant anonymes (IDA) ;
- mise à jour de la table locale (PLT) intégrant les nouvelles informations modificatives ;
- chiffrement de la table locale (PLT) au moyen de la clé de chiffrement.

9. Procédé selon la revendication 8 **caractérisé en ce que** l'agent d'une dispositif de stockage (20) quelconque appartenant à un même groupe ou organisme effectue les étapes suivantes pour vérifier l'opportunité d'une mise à jour de la table locale de liens (PLT, 21);
- vérification (810) du mot de passe du titulaire du dispositif de stockage (20) considéré;
- vérification (820) de la présence du fichier comportant la clé de chiffrement et de la table de liens (PLT, 21);
- génération d'une requête (830) transmise vers ledit premier serveur DMA (300) en vue de l'obtention de la liste des identifiants anonymes stockés sur ce serveur ;
- identification (840) de la liste des identifiants anonymes (IDA) téléchargés depuis ledit premier serveur DMA (300) ;
- déchiffrement (850) de la table de liens locales (PLT, 21) ;
- comparaison (860) de la liste des identifiants anonymes téléchargés avec celle stockée dans la table (PLT, 21) et, dans le cas d'une non concordance (870) ;
- génération d'une requête (880) à destination dudit second serveur (TSB, 400) dans le but de télécharger les informations nominatives qui y sont stockées temporairement ;
- mise à jour (890) de la table locale de liens (PLT, 21) au moyens des informations téléchargées depuis ledit second serveur (TSB, 400) ;
- vérification (890) de la mise à jour de tous les dispositifs de stockage d'un même groupe et purge, le cas échéant, des données stockées sur ledit second serveur (TSB, 400).

10. Procédé selon la revendication 9 **caractérisé en ce qu'**il comporte un outre la mise en oeuvre d'un serveur administrateur (200) permettant la gestion des licences et des purges dudit second serveur (TSB, 400) lorsque toutes la mise à jour de tous les dispositifs de stockage (20) appartenant à un même groupe ont été effectués.
